# EUROPEAN PATENT SPECIFICATION

(11) **EP 4 251 607 B1**
(45) Date of publication and mention of the grant of the patent: **12.02.2025**
(21) Application number: 21790984.5
(22) Date of filing: 27.08.2021
(51) Int. Cl.: C07C 269/06, C07C 271/16

(54) **METHOD FOR THE PREPARATION OF OLIGOCARBAMATES WITH A DEFINED MONOMERIC SEQUENCE**
VERFAHREN ZUR HERSTELLUNG VON OLIGOCARBAMATEN MIT DEFINIERTEM MONOMERABLAUF
PROCÉDÉ DE PRÉPARATION D'OLIGOCARBAMATES PRÉSENTANT UNE SÉQUENCE MONOMÈRE DÉFINIE

(30) Priority: 30.11.2020 PL 43616520
(43) Date of publication of application: 04.10.2023
(73) Proprietor: Siec Badawcza Lukasiewicz - PORT Polski Osrodek Rozwoju Technologii, 54-066 Wroclaw (PL)
(72) Inventor: SZWEDA, Róza, 54-106 Wroclaw (PL); WALENCIK, Paulina Katarzyna, 66-002 Zielona Góra (PL); TURSKI, Mateusz Boleslaw, 66-415 Santocko (PL); CWYNAR, Pawel, 54-130 Wroclaw (PL)
(74) Representative: JWP Patent & Trademark Attorneys
(86) International application number: PCT/IB2021/057872
(87) International publication number: WO 2022/112868

(56) References cited:
- EP-A1- 3 243 857

## Description

The subject matter of the invention is a method for the preparation of oligocarbamates with a defined monomeric sequence by one-pot synthesis.

One-pot synthesis is understood as a strategy in which the synthesis initiating substrate (initiator) is subjected to subsequent chemical reactions in a controlled manner, in one reactor, without intermediate products being isolated between individual reactions. In other words, it is multi-stage synthesis performed in one pot, without purifying the products in its various steps.

The currently used methods of oligo- and polycarbamate synthesis are methods of stepwise polymerisation (see, for example: Akindoyo, J. O., Beg, M. D. H., Ghazali, S., Islam, M. R., Jeyaratnam, N. & Yuvaraj, A. R. RSC Advances 2016, Vol. 6 114453-114482 and Chen, Z., Hadjichristidis, N., Feng, X. & Gnanou, Y. Macromolecules 2017, 50, 2320-2328), but these methods make it impossible to control the monomeric sequence. The oligo- and polycarbamates obtained by polymerisation are characterised by a heterogeneous structure and distribution of molar masses. The only method known so far for synthesizing homogeneous oligo- and polycarbamates with a controlled monomeric sequence is iterative synthesis on a support or in a solution. In the method on a support, synthesis is performed on a hydroxy-functionalised support with two reactions being employed: (i) activation of the alcohol group with N,N'-disuccinimidyl carbonate, and (ii) a chemoselective aminoalcohol coupling reaction resulting in a carbamate bond. On the other hand, all methods of synthesis of oligo- and polycarbamates in a solution described so far require purification between their steps.

The invention described in application EP3243857A1 relates to polyurethanes with a particular sequence and methods for their preparation. Said application discloses novel synthetic polyurethanes having different functional groups with unique, defined structures, as well as a method for preparing these uniform polyurethanes with a specific sequence. In general, according to the invention described therein, the synthesis of polyurethanes comprises (i) dissolving N,N'-disuccinimidyl carbonate in dry acetonitrile and adding triethylamine to the solution, the reaction takes place at 60°C, then transferring to a solid phase extraction tube and rinsing with N,N-dimethylformamide, and step (ii) of coupling which comprises adding a solution of triethylamine and aminoalcohol in dry N,N-dimethylformamide to an extraction tube containing a solid support activated with N,N'-disuccinimidyl carbonate. Steps (i) and (ii) are repeated an appropriate number of times to obtain an oligocarbamate chain of a desired length. In each step (ii), the molecular structure of the block which builds the aminoalcohol may be different to synthesise oligomers with a controlled sequence.

A similar method employing a solid support is described in Gunay, U. S., Petit, B. E., Karamessini, D., Al Ouahabi, A., Amalian, J. A., Chendo, C., Bouquey, M., Gigmes, D., Charles, L. & Lutz, J. F. Chem 2016, 1, 114-126. These methods, however, are based on synthesis on a solid support which has a number of constraints: (1) small scale of synthesis, (2) the need to use an excess of reagents, (3) high costs of a support, (4) the inability to monitor synthesis by traditional chromatographic methods, (5) the need to use a large excess of solvents to purify the product after each step. These constraints do not allow oligo- and polycarbamates to be obtained on an industrial scale, which makes it impossible to use these syntheses in industry.

All described methods of synthesis in a solution require purification between steps (J. Li, M. Leclercq, M. Fossepré, M. Surin, K. Glinel, A. M. Jonas, A. E. Fernandes, Polym. Chem. 2020, 11, 4040-4046; Hoff, E. A., De Hoe, G. X., Mulvaney, C. M., Hillmyer, M. A. & Alabi, C. A. J. Am. Chem. Soc. 2020, 142, 6729-6736). The necessity for purification after each step using chromatography makes synthesis very complicated, increases the time and cost of synthesis, requires the use of an additional portion of solvents and materials needed to isolate products and generates efficiency losses. Recently, a scalable method of synthesizing oligocarbamate-based gels with a controlled monomeric sequence has been described in the literature. The synthesis is performed in a solution using vanillin carbonate as an activator (Hoff, E. A., De Hoe, G. X., Mulvaney, C. M., Hillmyer, M. A. & Alabi, C. A. J. Am. Chem. Soc. 2020, 142, 6729-6736). However, this method makes it possible to control the monomeric sequence of the obtained macromolecules only within the side group of nitrogen substituents. The method involves purification by extraction after each step.

The aim of the invention was to develop a method for the synthesis of oligocarbamates with a defined monomeric sequence that would overcome the said disadvantages of the methods known from the prior art, i.e. difficult scalability, the necessity for purification after each step, a complicated synthesis protocol, the necessity to use a support, high costs of synthesis and high consumption of solvents and reagents.

The subject matter of the invention is a method for the preparation of oligocarbamates with a defined monomeric sequence of the general formula 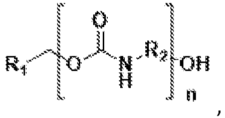 characterised in that the multistage synthesis is performed in one reaction pot without the necessity for purification between successive reactions, and the synthesis is driven by the addition of fresh portions of the N,N'-disuccinimidyl carbonate activator or a monomer in the form of HO-R₂-NH₂ aminoalcohol, after complete conversion of the previous step has been confirmed, the method comprising the following steps:
i. initiation which comprises the activation of 1 equiv. of alcohol dissolved in dry acetonitrile - a solution of 0.1-1 M with 3-12 equiv. of dry pyridine added - by the addition of an activator, being 1,2-2,1 equiv. of N,N'-disuccinimidyl carbonate, to the mixture and performing the reaction at room temperature until complete activation of alcohol, wherein the R₁-OH alcohol is an aromatic primary or secondary alcohol not containing free nucleophilic groups in its structure;
ii. at least one propagation of the oligomer chain by the addition of a portion of 1-2 equiv. of the first monomer in the form of HO-R ₂-NH₂ alcohol to 1 equiv. of the activated alcohol of step i. and performing the reaction until complete conversion of the activated alcohol, and then the addition of a further portion of the activator, being 1,2-2,1 equiv. of N,N'-disuccinimidyl carbonate, to activate the hydroxyl group of the growing macromolecule, wherein the substituent R₂ is selected from the group comprising a linear or branched C3-C8 alkyl and a C3-C8 alkyl having at least one aryl, composed of 3-8 carbon atoms and not containing free nucleophilic groups in its structure;
iii. repeating the propagation step until the desired oligocarbamate sequence is achieved.

Preferably, R₁-OH alcohol is selected from the group comprising benzyl alcohol, 3-[(Boc-aminomethyl)phenyl]methanol and 2-Boc-amino-3-phenylol-1-propanol.

Preferably, HO-R ₂-NH₂ aminoalcohol is selected from the group comprising 3-amino-1-propanol, (*S*)-(+)-1-amino-2-propanol, *(R)*-(+)-1-amino-2-propanol, *(S)*-2-amino-1-propanol, *(R)*-2-amino-1-propanol and 3-(aminomethyl)phenylmethanol.

Preferably, the reactions in steps (i.-iii.) are performed under inert atmosphere. More preferably, the inert atmosphere is nitrogen or argon atmosphere.

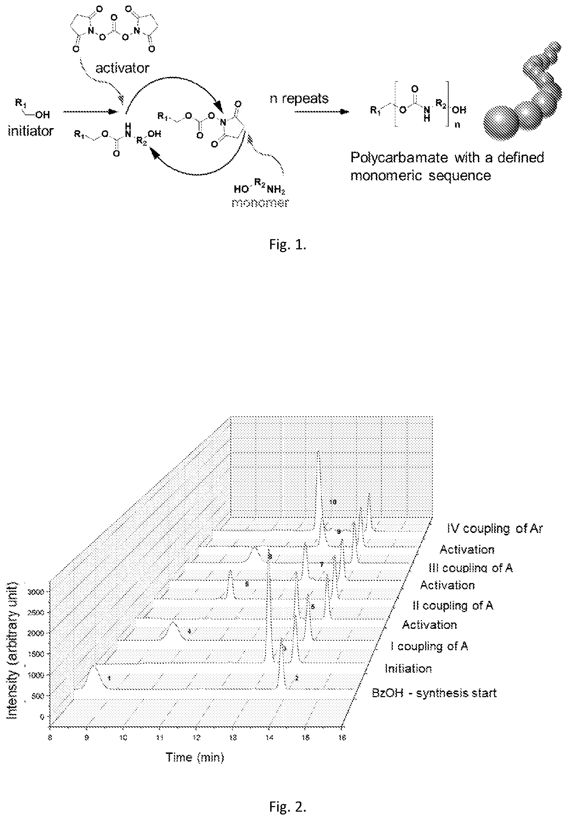

The method for the preparation of oligocarbamates with a defined monomeric sequence according to the invention is shown in Fig. 1. The method makes it possible to obtain homogeneous oligocarbamates with a defined monomeric sequence of the general formula shown in Scheme 1 A,

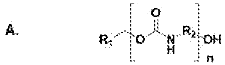

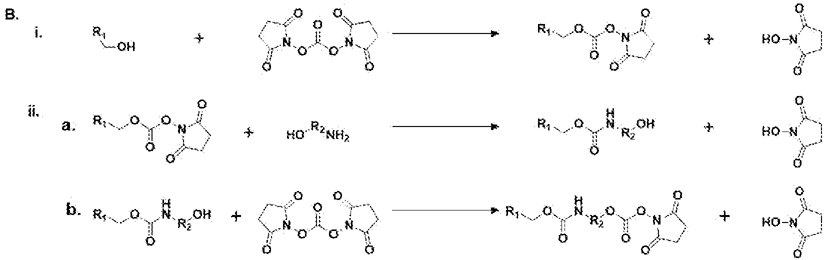

Scheme 1. The reactions employed to prepare oligocarbamates with a defined monomeric sequence according to the developed invention.
and is characterised in that it comprises the following steps:
   i. initiation by the activation of the alcohol with N,N'-disuccinimidyl carbonate according to the reaction (Scheme 1 B (i.)),
      wherein R₁ is an aromatic substituent having no nucleophilic substituents and reactive functional groups in its structure, an aromatic substituent with a Boc-protected amino group, with, e.g., benzyl alcohol, 3-[(Boc-aminomethyl)phenyl]methanol and 2-Boc-amino-3-phenylol-1-propanol possible to be used as the initiator.
   ii. propagation of the oligomer chain (Scheme 1 B (ii.)) by a. chemoselective coupling of the activated alcohol with the amino group of the aminoalcohol and b. activation of the hydroxyl group of the growing oligomer with N,N'-disuccinimidyl carbonate,
wherein the aminoalcohol employed cannot contain nucleophilic groups and reactive functional groups in its structure, where R₂ is an alkyl framework or aromatic framework, with the examples of monomers summarised in Table 1.

**Table 1 Examples of aminoalcohol monomers**

| **Structure** | **Names** | **Acronym** |
|---|---|---|
| 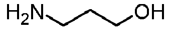 | 3-amino-1-propanol | **A** |
| 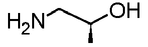 | (S)-(+)-1-amino-2-propanol | **C_{S}** |
| 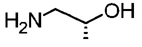 | (R)-(+)-1-amino-2-propanol | **C_{R}** |
| 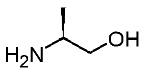 | (S)-2-amino-1-propanol | **D_{S}** |
| 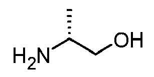 | (R)-2-amino-1-propanol | **D_{R}** |
| 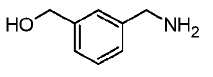 | 3-(aminomethyl)phenylmethanol | **Ar** |

Preferably, R₁ is an aromatic substituent having no nucleophilic substituents and reactive functional groups in its structure, an aromatic substituent with a Boc-protected amino group, with, e.g., benzyl alcohol, 3-[(Boc-aminomethyl)phenyl]methanol and 2-Boc-amino-3-phenylol-1-propanol possible to be used as the initiator.

Preferably, R₂ is an alkyl of a various structure, e.g. a linear structure (-CH₂-CH₂-CH₂-), a non-linear structure (-CH₂-CH(CH₃)- or an alkyl having at least one aryl (-CH₂-C₆H₄-CH₂-).

Preferably, the aminoalcohol is selected from the group comprising the aminoalcohols shown in Table 2, including 3-aminopropan-1-ol, (*S*)-(+)-2-aminopropan-1-ol, (R)-2-aminopropan-1-ol, m-(aminomethyl)benzyl alcohol.

Additionally, preferably, the reagents in steps (i.-ii.) are used in amounts of from equimolar to double molar excess with respect to the amount of the initiator used.

Preferably, the reactions in steps (i.-ii.) are performed under inert atmosphere. More preferably, the inert atmosphere is nitrogen or argon atmosphere.

Furthermore, preferably, the N-hydroxysuccinimide by-product is removed after synthesis by washing with water.

Preferably, the reactions in steps (i.-ii.) are performed in the presence of pyridine.

Preferably, the reactions in steps (i.-ii.) are performed at room temperature.

The developed method of one-pot synthesis begins with the reaction of i. activation of an alcohol with an active carbonate, followed by a chemoselective reaction of the resulting product with the amino group of aminoalcohol ii. (in particular aminoalcohols with primary and secondary amine/alcohol). The free hydroxyl group of the resulting product is activated by the addition of a fresh portion of the activator. After complete conversion of the hydroxyl group to carbonate, synthesis is continued by the addition of a portion of another monomer. The two reactions ii. are repeated cyclically until the desired oligomer sequence is obtained. Importantly, the monomer/activator is added after complete conversion of the previous step is confirmed. Whether the conversion of the activator/monomer is complete can be verified, e.g., by HPLC chromatography with a reversed-phase C18 column. Each addition of another portion of monomer makes it possible to control the order of monomers in the chain. The steps of activation and chemoselective coupling of alcohol are repeated until an oligomer of a desired monomeric sequence is obtained. The reactions are performed in one reaction pot and the course of subsequent steps is driven by the addition of a new portion of activator/monomer reagents. Each step of aminoalcohol activation/coupling generates the N-hydroxysuccinimide by-product, which is removed after synthesis by washing with water. In the disclosed method, it is critical to perform alcohol activation and coupling reactions until complete conversion. When calculating the amounts of reagents, the purity of the reagents used and the conversion of substrates should be taken into account. Depending on the monomers used and the target oligomer sequence, some reactions require the use of excesses of reagents. The excesses needed are calculated based on the amount of the unreacted substrates based on the chromatographic analysis, which means that a suitable amount of moles is added necessary for complete conversion. High purity (e.g. dry) solvents should be used for synthesis and, if possible, the reaction should be performed under inert atmosphere (e.g. nitrogen or argon atmosphere).

A distinctive feature of the proposed method of oligocarbamate synthesis according to the invention is that the synthesis is conducted in one pot and no purification is needed, e.g., no extraction or precipitation after each step of synthesis, no support that would limit the scale of synthesis or require the use of a large excess of reagents/solvents. The main element of the method for preparation of oligocarbamates (oligourethanes) with a specific sequence is a one-pot synthesis driven by successive portions of (activator/monomer) reagents. The developed method of synthesis has many advantages over the methods currently used: (1) it is scalable (Example O2-O2', O6-O6'), (2) it limits the use of organic solvents, (3) it does not require the use of a large excess of reagents, (4) it does not require purification after each synthesis step and (5) it does not require the use of a support. Given points (2), (3), (4) and (5), the developed method will significantly reduce the cost of synthesis compared to the methods currently used. The oligocarbamates obtained by the method are characterised by a defined chemical structure, i.e. a defined sequence of monomers in the chain, defined end groups, which can be subjected to further modifications, and no molar mass dispersions or, in other words, a homogeneous structure (Examples 01-07).

The developed method consists in a reaction sequence of activation and chemoselective coupling of aminoalcohol monomers in one reaction pot, according to the scheme shown in Fig. 1. The distinctive features and undoubted advantages of the method of the present invention are also:
(1) Complete control of the monomer sequence in the chain;
(2) Control of the end groups of the oligomer;
(3) Conducting synthesis in one pot, which limits the amount of organic solvents;
(4) No necessity to use a large excess of reagents as is the case with the methods of synthesis on a support;
(5) No necessity for purification at every step of synthesis;
(6) Scalability of the method;
(7) Easy monitoring of the synthesis using chromatography;
(8) Lower costs of synthesis compared to the methods using a support;
(9) The method of synthesis being easily automatized using commercially available synthesis robots, such as Chemspeed, since the synthesis protocol is very simple and consists only in appropriate dosing of reagents;
(10) The oligomers obtained by the method can be employed as substrates for the synthesis of higher molecular weight polymers, in particular the oligomers obtained using an initiator being the aminoalcohol with an amino group blocked by tert-butyl carbamate;
(11) Control of the chiral centre sequence in the main chain by selection of appropriate monomers.

The subject matter of the invention is shown in the attached drawing, in which:
Fig. 1. shows an illustrative scheme of oligocarbamate synthesis according to the present invention and the principle of operation of this fuel-driven, one-pot synthesis of oligocarbamates with a defined monomeric sequence.
Fig. 2. shows the synthesis of the Bz-AAAAr (O1) oligomer being monitored by reversed phase liquid chromatography. The chromatograms were recorded at 220 nm.

### Embodiments

### Embodiments 01-07

| | **Oligocarbamates^{[a]}** | **Molar mass^{[b]}** | **m/z^{[c]}** | **Purity [%]^{[d]}** | **Yield [%]^{[e]}** | **Scale [g]^{[f]}** |
|---|---|---|---|---|---|---|
| **O1** | Bz-AAAAr | 574.26 | 575.27 | 92.3 | 90.0 | **0.385** |
| **O2** | Boc-ArAAAA | 641.33 | 642.34 | 99.6 | 75.4 | **3.798** |
| **O2'** | Boc-ArAAAA | | 642.34 | 98.0 (MeOH) | 71.9 | **0.154** |
| **O3** | Boc-PaDᵣAAA | 655.34 | 656.35 | 97.13 | 89.5 | **4.399** |
| **O4** | Boc-PaDₛAAA | 655.34 | 656.35 | 96.5 | 92.3 | **1.303** |
| **O5** | Boc-PaCₛCₛCₛCₛ | 655.34 | 656.35 | 96.0 | 94.6 | **2.268** |
| **O6** | Boc-ArDₛAAA | 641.33 | 642.34 | 96.2 | 85.6 | **1.356** |
| **O6'** | Boc-ArDₛAAA Boc-ArDₛAAA | 641.33 | 642.34 | 97.0 (MeOH) | 65.2 | **0.164** |
| **O7** | Boc-PaDrDsDrDs | 655.34 | 656.35 | 96.7 | 93.6 | **1.106** |

| | | | | | | |
|---|---|---|---|---|---|---|
| *^{a}the abbreviations stand for oligocarbamates built from aminoalcohol monomers linked by a carbamate bond: 3-amino-1-propanol (A), 3-(aminomethyl)phenylmethanol (Ar), benzyl alcohol (Bz), (S)-1-amino-2-propanol (Cₛ), (S)-2-amino-1-propanol (Dₛ), (R)-2-amino-1-propanol (Dᵣ), (S)-2-amino-3-phenyl-1-propanol (Pa); ^{b}monoisotopic mass; ^{c}result of LC-MS analysis; "calculated based on the HPLC chromatogram; ^{e}yields calculated for crude products isolated by extraction; ^{f}amount of product obtained.* | | | | | | |

### General description of the experimental procedure for the synthesis of 01-07 oligocarbamates

*Initiation (Scheme 1, B i.):* Alcohol (1 equiv.) was dissolved in dry acetonitrile (0.1-1 M solution). Dry pyridine (Pyr, 3-12 equiv.) and anisole standard (10 mol% relative to alcohol) were then added to the solution, and then the N,N'-disuccinimidyl carbonate activator (DSC, 1.2 equiv.) was added to the mixture. The reaction was performed at room temperature until complete activation of alcohol.

*Propagation (Scheme 1, B ii.):* To the activated alcohol (1 equiv.) was added a portion of the first monomer: (1 equiv.). The reaction was performed until complete conversion of the activated alcohol. Then another portion of the N,N'-disuccinimidyl carbonate activator (DSC, 1.2 equiv.) was added to activate the hydroxyl group of the growing macromolecule. After complete activation of the hydroxyl group, the coupling reaction of another monomer (1 equiv.) was performed. According to the above protocol, successive reactions of hydroxyl group activation by adding DSC activator (DSC, 1.2 equiv.) and monomer coupling were repeated until the desired oligomer sequence was obtained. All reactions were performed at room temperature and under nitrogen atmosphere and their progress was controlled by HPLC chromatography. In the disclosed method, it is critical to perform alcohol activation and monomer coupling reactions until complete conversion of substrates. Note: High purity (e.g. dry) solvents were used for synthesis and the reactions were performed under inert atmosphere (e.g. nitrogen or argon atmosphere). Depending on the monomers used and the target oligomer sequence, some reactions require the use of excesses of reagents. The excesses needed are calculated based on the amount of the unreacted substrates determined using chromatography, which means that a suitable amount of moles is added necessary for complete conversion. This amount is set experimentally for each sequence based on synthesis monitoring. The table below provides the exact amounts of reagents used and the excesses of reagents used during 01-07 syntheses.

| **Synthesis of O1 Bz-AAAAr** | | |
|---|---|---|
| **Synthesis step** | **Amount of reagents** | **Additional** portion of **reagents** |
| **I activation** | DSC (284.3 mg 1.2 mol equiv.) | - |
| **I coupling** | A (72.1 µL, 1 mol equiv.) | - |
| **II activation** | DSC (284.3 mg, 1.2 mol equiv.) | + 48.7 mg (0.2 mol equiv.) |
| **II coupling** | A (72.1 µL, 1 mol equiv.) | - |
| **III activation** | DSC (284.3 mg, 1.2 mol equiv.) | + 31.0 mg (0.1 mol equiv.) |
| **III coupling** | A (72.1 µL, 1 mol equiv.) | - |
| **IV activation** | DSC (284.3 mg, 1.2 mol equiv.) | - |
| **IV coupling** | Ar (130 mg, 1 mol equiv.) | - |

| **Synthesis of O2 Boc-ArAAAA** | | |
|---|---|---|
| **Synthesis step** | **Amount of reagents** | **Additional portion of reagents** |
| **I activation** | DSC (2.5279 g, 1.2 mol equiv.) | - |
| **I coupling** | A (629 µL, 1 mol equiv.) | - |
| **II activation** | DSC (2.5279 g, 1.2 mol equiv.) | - |
| **II coupling** | A (629 µL, 1 mol equiv.) | - |
| **III activation** | DSC (2.5279 g 1.2 mol equiv.) | + 84.0 mg (0.04 mol equiv.) |
| **III coupling** | A (629 µL, 1 mol equiv.) | + 100 µL (0.2 mol equiv.) |
| **IV activation** | DSC (2.5279 g 1.2 mol equiv.) | + 1.1 g (0.5 mol equiv.) |
| **IV coupling** | A (629 µL, 1 mol equiv.) | + 400 µL (0.6 mol equiv.) |

| **Synthesis of O2'** Boc-ArAAAA | | |
|---|---|---|
| **Synthesis step** | **Amount of reagents** | **Additional portion of reagents** |
| **I activation** | DSC (225.7 mg, 2.1 mol equiv.) | - |
| **I coupling** | A (57 µL, 1.7 mol equiv.) | - |
| **II activation** | DSC (225.7 mg, 2.1 mol equiv.) | - |
| **II coupling** | A (57 µL, 1.7 mol equiv.) | +8.1 µL (0.3 mol equiv.) |
| **III activation** | DSC (225.7 mg, 2.1 mol equiv.) | - |
| **III coupling** | A (57 µL, 1.7 mol equiv.) | - |
| **IV activation** | DSC (225.7 mg, 2.1 mol equiv.) | - |
| **IV coupling** | A (57 µL, 1.7 mol equiv.) | - |

| **Synthesis of O3 Boc-PaDᵣAAA** | | |
|---|---|---|
| **Synthesis step** | Amount of reagents | **Additional portion of reagents** |
| **I activation** | DSC (2.4507 g, 1.2 mol equiv.) | - |
| **I coupling** | Dᵣ (622 µL, 1 mol equiv.) | + 180 µL (0.3 mol equiv.) |
| **II activation** | DSC (2.4507 g, 1.2 mol equiv.) | - |
| **II coupling** | A (610 µL, 1 mol equiv.) | - |
| **III activation** | DSC (2.4507 g, 1.2 mol equiv.) | - |
| **III coupling** | A (610 µL, 1 mol equiv.) | + 180 µL (0.3 mol equiv.) |
| **IV activation** | DSC (2.4507 g 1.2 mol equiv.) | + 0.552 g (0.3 mol equiv.) |
| **IV coupling** | A (610 µL, 1 mol equiv.) | + 300 µL (0.5 mol equiv.) |

| **Synthesis of O4 Boc-PaDₛAAA** | | |
|---|---|---|
| **Synthesis step** | **Amount of reagents** | **Additional portion of reagents** |
| **I activation** | DSC (0.696 g, 1.2 mol equiv.) | - |
| **I coupling** | Dₛ (176 µL, 1 mol equiv.) | + 8.3µL (0.05 mol equiv.) |
| **II activation** | DSC (0.696 g, 1.2 mol equiv.) | - |
| **II coupling** | A (173 µL, 1 mol equiv.) | - |
| **III activation** | DSC (0.696 g, 1.2 mol equiv.) | + 49.5 mg (0.1 mol equiv.) |
| **III coupling** | A (173 µL, 1 mol equiv.) | + 16.2 µL (0.1 mol equiv.) |
| **IV activation** | DSC (0.696 g, 1.2 mol equiv.) | - |
| **IV coupling** | A (173 µL, 1 mol equiv.) | + 16.2 µL (0.1 mol equiv.) |

| **Synthesis of O5 Boc-PaCₛCₛCₛCₛ** | | |
|---|---|---|
| **Synthesis step** | **Amount of reagents** | **Additional portion of reagents** |
| **I activation** | DSC (1.2645 g, 1.2 mol equiv.) | - |
| **I coupling** | Cₛ (324 µL, 1 mol equiv.) | - |
| **II activation** | DSC (1.2645 g, 1.2 mol equiv.) | + 250 mg (0.2 mol equiv.) |
| **II coupling** | Cₛ (324 µL, 1 mol equiv.) | + 25.2 µL (0.1 mol equiv.) |
| **III activation** | DSC (1.2645 g 1.2 mol equiv.) | + 118 mg (0.1 mol equiv.) |
| **III coupling** | Cₛ (324 µL, 1 mol equiv.) | + 60 µL (0.2 mol equiv.) |
| **IV activation** | DSC (1.2645 g 1.2 mol equiv.) | + 416 mg (0.4 mol equiv.) |
| **IV coupling** | Cₛ (324 µL, 1 mol equiv.) | + 350 µL (1.1 mol equiv.) |

| **Synthesis of** O6_Boc-ArDₛAAA | | |
|---|---|---|
| **Synthesis step** | **Amount of** reagents | **Additional portion of reagents** |
| **I activation** | DSC (0.8514 g 1.2 mol equiv.) | - |
| **I coupling** | Dₛ (216 µL, 1 mol equiv.) | - |
| **II activation** | DSC (0.8514 g 1.2 mol equiv.) | +350.5 mg (0.5 mol equiv.) |
| **II coupling** | A (212 µL, 1 mol equiv.) | + 50 µL (0.2 mol equiv.) |
| **III activation** | DSC (0.8514 g, 1.2 mol equiv.) | +693.6 mg (1.0 mol equiv.) |
| **III coupling** | A (212 µL, 1 mol equiv.) | + 60 µL (0.3 mol equiv.) |
| **IV activation** | DSC (0.8514 g, 1.2 mol equiv.) | +923.5 mg (1.3 mol equiv.) |
| **IV coupling** | A (212 µL, 1 mol equiv.) | + 80 µL (0.4 mol equiv.) |

| **Synthesis of 06'** Boc-ArDₛAAA | | |
|---|---|---|
| **Synthesis step** | **Amount of reagents** | **Additional portion of reagents** |
| **I activation** | DSC (256.4 mg 2.1 mol equiv.) | - |
| **I coupling** | Dₛ (64.9 µL, 1.7 mol equiv.) | - |
| **II activation** | DSC (256.4 mg 2.1 mol equiv.) | - |
| **II coupling** | A (63.7 µL, 1.7 mol equiv.) | - |
| **III activation** | DSC (256.4 mg 2.1 mol equiv.) | - |
| **III coupling** | A (63.7 µL, 1.7 mol equiv.) | - |
| IV activation | DSC (256.4 mg 2.1 mol equiv.) | - |
| **IV coupling** | A (63.7 µL, 1.7 mol equiv.) | - |

| **Synthesis of O7_Boc-PaDᵣDₛDᵣDₛ** | | |
|---|---|---|
| **Synthesis step** | **Amount of reagents** | **Additional portion of reagents** |
| **I activation** | DSC (0.5536 g, 1.2 mol equiv.) | - |
| **I coupling** | Dᵣ (140.5 µL, 1 mol equiv.) | - |
| **II activation** | DSC (0.5536 g 1.2 mol equiv.) | - |
| **II coupling** | Dₛ (140.2 µL, 1 mol equiv.) | - |
| **III activation** | DSC (0.5536 g 1.2 mol equiv.) | +202.7 mg (0.4 mol equiv.) |
| **III coupling** | Dᵣ (140.5 µL, 1 mol equiv.) | + 32 µL (0.2 mol equiv.) |
| **IV activation** | DSC (0.5536 g, 1.2 mol equiv.) | + 332 mg(0.7 mol equiv.) |

After completion of synthesis, the solvent was evaporated under reduced pressure (40°C). The product was dissolved in ethyl acetate and extracted with an aqueous NaCl solution to remove the resulting N-hydroxysuccinimide by-product. The organic phase containing the product was dried with anhydrous Na₂SO₄ and filtered. The solvent was then evaporated and the product was dried under reduced pressure (40°C).

*Characteristic:* HPLC chromatography was employed to monitor the progress of all reactions. Conversions were calculated against the anisole standard. The purity of the product was calculated based on the HPLC chromatogram at 220 nm. The reaction yield for the crude product isolated by extraction was calculated by gravimetry. The product structure, including the sequence, was confirmed by LC-MS analysis with fragmentation and ¹H NMR. The homogeneous structure and the lack of molar mass distribution were confirmed by GPC chromatography.

## Claims

1. Method for the preparation of oligocarbamates with a defined monomeric sequence of a general formula 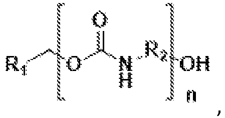 **characterised in that** the multistage synthesis is performed in one reaction pot without the necessity for purification between successive reactions, and the synthesis is driven by the addition of fresh portions of the N,N'-disuccinimidyl carbonate activator or a monomer in the form of HO-R₂-NH₂ aminoalcohol, after complete conversion of the previous step has been confirmed, the method comprising the following steps:
i. initiation which comprises the activation of 1 equiv. of alcohol dissolved in dry acetonitrile - a solution of 0.1-1 M with 3-12 equiv. of dry pyridine added - by the addition of an activator, being 1.2-2.1 equiv. of N,N'-disuccinimidyl carbonate, to the mixture and performing the reaction at room temperature until complete activation of alcohol, wherein the R₁-OH alcohol is an aromatic primary or secondary alcohol not containing free nucleophilic groups in its structure;
ii. at least one propagation of the oligomer chain by the addition of a portion of 1-2 equiv. of the first monomer in the form of HO-R₂-NH₂ aminoalcohol to 1 equiv. of the activated alcohol of step (i.) and performing the reaction until complete conversion of the activated alcohol, and then the addition of a further portion of the activator, being 1.2-2.1 equiv. of N,N'-disuccinimidyl carbonate, to activate the hydroxyl group of the growing macromolecule, wherein the substituent R₂ is selected from the group comprising a linear or branched C3-C8 alkyl and a C3-C8 alkyl having at least one aryl, composed of 3-8 carbon atoms and not containing free nucleophilic groups in its structure;
iii. repeating the propagation step until the desired oligocarbamate sequence is achieved.

2. The method according to claim 1, **characterised in that** R₁-OH alcohol is selected from the group comprising benzyl alcohol, 3-[(Boc-aminomethyl)phenyl]methanol and 2-Boc-amino-3-phenylol-1-propanol.

3. The method according to claim 1, **characterised in that** HO-R₂-NH₂ aminoalcohol is selected from the group comprising 3-amino-1-propanol, (*S*)-(+)-1-amino-2-propanol, *(R)*-(+)-1-amino-2-propanol, *(S)*-2-amino-1-propanol, *(R)*-2-amino-1-propanol and 3-(aminomethyl)phenylmethanol.

4. The method according to claim 1, **characterised in that** the reactions in steps (i.-iii.) are performed under inert atmosphere.

5. The method according to claim 4, **characterised in that** the inert atmosphere is nitrogen or argon atmosphere.

## Patentansprüche

1. Verfahren zur Herstellung von Oligocarbamaten mit einer definierten monomeren Sequenz einer allgemeinen Formel 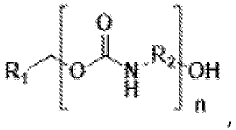 **dadurch gekennzeichnet, dass** die mehrstufige Synthese in einem Reaktionstopf ohne die Notwendigkeit für Reinigung zwischen sukzessiven Reaktionen durchgeführt wird, und die Synthese durch die Zugabe von frischen Portionen des N,N'-Disuccinimidylcarbonataktivators oder eines Monomers in Form von einem HO-R₂-NH₂-Aminoalkohol angetrieben wird, nachdem ein vollständiger Umsatz des vorherigen Schritts bestätigt wurde, wobei das Verfahren die folgenden Schritte umfasst:
i. Initiierung, die die Aktivierung von 1 Äquiv. eines in trockenem Acetonitril gelösten Alkohols - einer 0,1-1 M Lösung mit 3-12 zugegebenen Äquiv. trockenem Pyridin - durch die Zugabe eines Aktivators, bei dem es sich um 1,2-2,1 Äquiv. eines N,N'-Disuccinimidylcarbonats handelt, zu der Mischung und Durchführung der Reaktion bei Raumtemperatur bis zu einer vollständigen Aktivierung eines Alkohols umfasst, wobei es sich bei dem R₁-OH-Alkohol um einen keine freien nukleophilen Gruppen in seiner Struktur enthaltenden aromatischen primären oder sekundären Alkohol handelt;
ii. mindestens eine Propagierung der Oligomerkette durch die Zugabe einer Portion von 1-2 Äquiv. des ersten Monomers in Form von einem HO-R₂-NH₂-Aminoalkohol zu 1 Äquiv. des aktivierten Alkohols des Schritts (i.) und Durchführung der Reaktion bis zu einem vollständigen Umsatz des aktivierten Alkohols und dann die Zugabe einer weiteren Portion des Aktivators, bei dem es sich um 1,2-2,1 Äquiv. eines N,N'-Disuccinimidylcarbonats handelt, um die Hydroxylgruppe des wachsenden Makromoleküls zu aktivieren, wobei der R2-Substituent ausgewählt ist aus der Gruppe umfassend ein lineares oder verzweigtes C3-C8-Alkyl und ein C3-C8-Alkyl aufweisend mindestens ein Aryl, das aus 3-8 Kohlenstoffatomen zusammengesetzt ist und keine freien nukleophilen Gruppen in seiner Struktur enthält;
iii. Wiederholung des Propagierungsschritts, bis die gewünschte Oligocarbamatsequenz erreicht ist.

2. Das Verfahren nach Anspruch 1, **dadurch gekennzeichnet, dass** der R₁-OH-Alkohol ausgewählt ist aus der Gruppe umfassend Benzylalkohol, 3-[(Boc-aminomethyl)phenyl]methanol und 2-Boc-amino-3-phenylol-1-propanol.

3. Das Verfahren nach Anspruch 1, **dadurch gekennzeichnet, dass** der HO-R₂-NH₂-Aminoalkohol ausgewählt ist aus der Gruppe umfassend 3-Amino-1-propanol, (*S*)-(+)-1-Amino-2-propanol, (*R*)-(+)-1-Amino-2-propanol, (*S*)-2-Amino-1-propanol, (*R*)-2-Amino-1-propanol und 3-(Aminomethyl)phenylmethanol.

4. Das Verfahren nach Anspruch 1, **dadurch gekennzeichnet, dass** die Reaktionen in Schritten (i.-iii.) unter inerter Atmosphäre durchgeführt werden.

5. Das Verfahren nach Anspruch 4, **dadurch gekennzeichnet, dass** es sich bei der inerten Atmosphäre um eine Stickstoff- oder Argonatmosphäre handelt.

## Revendications

1. Procédé pour préparation des oligocarbamates présentant une séquence monomère définie de formule générale 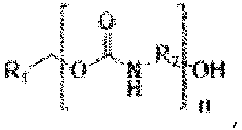 **caractérisé en ce que** la synthèse multi-étapes est effectuée dans un réacteur à réaction unique sans nécessité de purification entre les réactions successives, et la synthèse est actionnée par l'adjonction des nouveaux parts d'un activateur de carbonate de N,N'-disuccinimidyle ou un monomère sous forme d'un aminoalcool de formule HO-R₂-NH₂, après la transformation complet de l'étape précédente était confirmée, le procédé comprenant les étapes suivantes :
i. amorçage qui comprend l'activation d'un équivalent d'alcool dissolu dans l'acétonitrile sec - une solution de 0.1-1 M avec 3-12 équiv. de la pyridine sèche ajouté - par l'adjonction d'un activateur, étant 1.2-2.1 équiv. de carbonate de N,N'-disuccinimidyle, au mélange et en effectuant la réaction à température ambiante jusqu'à l'activation complet d'alcool, un alcool de formule R₁-OH étant un alcool aromatique primaire ou secondaire ne contenant pas de groupes nucléophiles libres dans leur structure ;
ii. au moins une étape de propagation de la chaîne oligomère par l'adjonction d'un part de 1-2 équiv. d'une première monomère sous forme d'un aminoalcool de formule HO-R₂-NH₂ à 1 équiv. d'un alcool activé d'étape (i.) et en effectuant la réaction jusqu'à la transformation complet d'alcool activé, et ensuite l'adjonction d'un part ultérieur d'activateur, étant 1.2-2.1 équiv. de carbonate de N,N'-disuccinimidyle, pour activer le groupe hydroxyle d'une macromolécule croissante, dans laquelle un substituant R₂ est sélectionné dans le groupe comprenant un alkyle en C3-C8 linéaire ou ramifié et un alkyle en C3-C8 présentant au moins un groupe aryle, composé de 3-8 atomes de carbone et ne contenant pas de groupes nucléophiles libres dans leur structure ;
iii. en répétant l'étape de propagation jusqu' à la séquence oligocarbamate souhaitée est obtenue.

2. Procédé selon la revendication 1, **caractérisé en ce qu'**un alcool de formule R₁-OH est sélectionné dans le groupe comprenant l'alcool benzylique, 3-[(Boc-aminométhyl)phényle]méthanol et 2-Boc-amino-3-phénylol-1-propanol.

3. Procédé selon la revendication 1, **caractérisé en ce qu'**un aminoalcool de formule HO-R₂-NH₂ est sélectionné dans le groupe comprenant 3-amino-1-propanol, (S)-(+)-1-amino-2-propanol, (R)-(+)-1-amino-2-propanol, (S)-2-amino-1-propanol, (R)-2-amino-1-propanol et 3-(aminométhyl)phénylméthanol.

4. Procédé selon la revendication 1, **caractérisé en ce que** les réactions dans les étapes (i.-iii.) sont effectuées sous une atmosphère inerte.

5. Procédé selon la revendication 4, **caractérisé en ce que** l'atmosphère inerte est une atmosphère d'azote ou d'argon.
